# FASCICULE DE BREVET EUROPEEN

(11) **EP 3 649 111 B1**
(45) Date de publication et mention de la délivrance du brevet: **25.08.2021**
(21) Numéro de dépôt: 18733636.7
(22) Date de dépôt: 02.07.2018
(51) Int. Cl.: C07D 233/34, C08F 8/30

(54) **MOLÉCULE POLYAROMATIQUE PORTANT UNE FONCTION OXYDE DE NITRILE**
POLYAROMATISCHES MOLEKÜL MIT EINER NITRILOXIDFUNKTION
POLYAROMATIC MOLECULE HAVING A NITRILE OXIDE FUNCTION

(30) Priorité: 04.07.2017 FR 1756300
(43) Date de publication de la demande: 13.05.2020
(73) Titulaire: Arkema France, 92700 Colombes (FR)
(72) Inventeur: SALIT, Anne-Frédérique, 63040 Clermont-Ferrand Cedex 09 (FR); FLEURY, Etienne, 63040 Clermont-Ferrand Cedex 09 (FR); IVANOV, Sergey, 63040 Clermont-Ferrand Cedex 09 (FR); JEAN-BAPTISTE-DIT-DOMINIQUE, François, 63040 Clermont-Ferrand Cedex 09 (FR); UGOLNIKOV, Oleg, 63040 Clermont-Ferrand Cedex 09 (FR)
(74) Mandataire: Casalonga
(86) Numéro de dépôt international: PCT/EP2018/067766
(87) Numéro de publication internationale: WO 2019/007881

(56) Documents cités:
- WO-A1-2012/007441
- WO-A1-2012/007684
- US-A1- 2011 054 134

## Description

La présente invention concerne le domaine des molécules associatives azotées comprenant au moins un motif les rendant susceptibles de s'associer entre elles ou avec une charge par des liaisons non covalentes, et comprenant une fonction capable de réagir avec un polymère comprenant des insaturations pour former une liaison covalente avec ledit polymère.

Plus précisément, la présente invention concerne les molécules porteuses d'une fonction oxyde de nitrile et d'une fonction imidazolidinone. La demande concerne également un procédé de synthèse de telles molécules.

Dans le domaine industriel, des mélanges de polymères avec des charges sont souvent utilisés. Pour que de tels mélanges présentent de bonnes propriétés, on recherche en permanence des moyens pour améliorer la dispersion des charges au sein des polymères. L'un des moyens pour parvenir à ce résultat est l'utilisation d'agents de couplage capables d'établir des interactions entre le polymère et la charge.

Des agents de couplage d'un polymère avec une charge comprenant des dipôles azotés sont décrits dans les documents publiés sous les numéros US7186845B2 et JP2008208163.

Ces documents décrivent la modification de polymères comprenant des motifs diéniques par des composés dipolaires azotés comprenant en outre un hétérocycle, ledit hétérocycle comprenant lui-même un atome d'azote, et un atome d'oxygène et/ou de soufre.

Plus particulièrement, les composés décrits sont des nitrones porteuses de fonction oxazoline, thiazoline comme par exemple la ((2-oxazolyl)-phényl-N-méthylnitrone) :

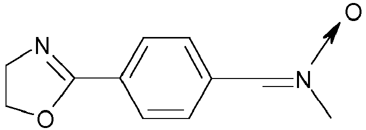

Lorsque des polymères diéniques sont amenés à réagir avec de tels composés, les polymères en résultant porteront les cycles oxazoline ou thiazoline.

Ces cycles présents sur le polymère sont susceptibles de réagir à leur tour, avec des fonctions de surface des charges, comme le noir de carbone ou la silice, avec lesquels les polymères sont mélangés. Cette réaction conduit à l'établissement de liaisons covalentes entre le polymère modifié par l'agent de couplage et la charge du fait de l'ouverture du cycle oxazoline ou thiazoline. En effet, comme cela est décrit dans le document US7186845B2, les cycles oxazolines et/ou thioazolines sont susceptibles de s'ouvrir en présence d'un nucléophile pouvant par exemple être présent à la surface de la charge.

L'établissement de telles liaisons covalentes présente néanmoins des inconvénients lors de la préparation de mélanges comprenant ces polymères modifiés par des agents de couplage avec des charges. En particulier, l'existence de ces liaisons covalentes précocement établies, entre le polymère et les charges, rend ces mélanges très visqueux à l'état non réticulé, ce qui rend difficile toutes les opérations préalables à la réticulation (vulcanisation) des formulations à base de caoutchouc, notamment la préparation des mélanges des composants, et leur mise en forme. Ces inconvénients ont un fort impact sur la productivité industrielle.

Il est donc souhaitable de proposer de nouvelles molécules ne présentant pas les inconvénients ci-dessus, c'est-à-dire des molécules qui soient capables après réaction avec un polymère et mélange avec une charge, de ne pas former de liaisons covalentes avec la charge et donc de ne pas provoquer une trop forte augmentation de la viscosité du mélange.

Ainsi, la demande de brevet WO 2012/007684 porte sur un composé comprenant au moins un groupement Q, et au moins un groupement A reliés entre eux par au moins et de préférence un groupe espaceur Sp dans lequel :
- Q comprend un dipôle contenant au moins et de préférence un atome d'azote ;
- A comprend un groupe associatif comprenant au moins un atome d'azote ;
- Sp est un atome ou un groupe d'atomes formant une liaison entre Q et A.

Lorsqu'un polymère greffé par un composé tel que défini ci-dessus est mélangé à des charges, celui-ci n'établit que des liaisons labiles avec les charges, ce qui permet d'assurer une bonne interaction polymère - charge, bénéfique pour les propriétés finales du polymère, mais sans les inconvénients qu'une trop forte interaction polymère - charge pourrait provoquer.

Un exemple d'un tel composé est l'oxyde de 2-[2-(2-oxoimidazolidin-1-yl)éthoxy]benzonitrile :

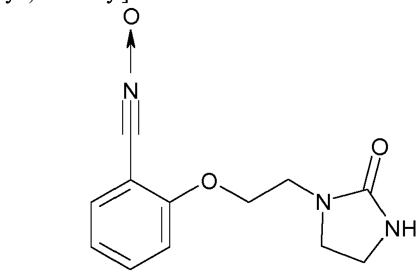

Par ailleurs, dans la demande WO 2016/207263, divers composés sont exemplifiés, dont notamment l'oxyde de 2,4,6-triméthyl-3-[2-(2-oxoimidazolidin-1-yl)éthoxy]benzonitrile :

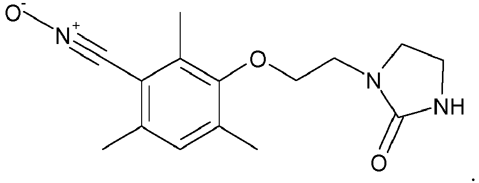

Il s'avère que les compositions de caoutchouc, utilisables notamment pour la fabrication de pneumatiques, comprenant cette molécule présentent des propriétés intéressantes en termes de rigidité, de contrainte à la rupture ou encore d'hystérèse. Néanmoins, il existe toujours un besoin constant de disposer de compositions de caoutchouc présentant des propriétés améliorées.

Dans ce contexte, la demanderesse a découvert qu'un composé polyaromatique particulier, une fois intégré dans des compositions de caoutchouc, utilisables notamment pour la fabrication de pneumatiques, conduisait à l'obtention d'excellentes propriétés de rigidité, de contrainte à la rupture et d'hystérèse.

L'invention a donc pour objet un composé de formule (I) suivante : 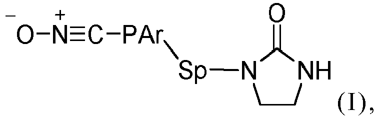 dans laquelle :
- PAr désigne un groupe polyaromatique comprenant au moins deux cycles hydrocarbures aromatiques condensés, chacun desdits cycles aromatiques condensés étant éventuellement substitué par une ou plusieurs chaînes carbonées, identiques ou différentes, indépendantes les unes des autres, aliphatiques ou aromatiques, linéaires, ramifiées ou cycliques, éventuellement substituées ou interrompues par un ou plusieurs hétéroatomes ;
- Sp représente un atome ou un groupe d'atomes.

Un autre objet de l'invention est un procédé de synthèse du composé de formule (I) selon l'invention.

On entend au sens de la présente invention par « chaîne carbonée » une chaîne comprenant un ou plusieurs atomes de carbone.

Dans la présente description, sauf indication expresse différente, tous les pourcentages (%) indiqués sont des % en masse. D'autre part, tout intervalle de valeurs désigné par l'expression "entre a et b" représente le domaine de valeurs allant de plus de a à moins de b (c'est-à-dire bornes a et b exclues) tandis que tout intervalle de valeurs désigné par l'expression "de a à b" signifie le domaine de valeurs allant de a jusqu'à b (c'est-à-dire incluant les bornes strictes a et b).

L'invention ainsi que ses avantages seront aisément compris à la lumière de la description et des exemples de réalisations qui suivent.

Les composés mentionnés dans la description peuvent être d'origine fossile ou biosourcés. Dans ce dernier cas, ils peuvent être, partiellement ou totalement, issus de la biomasse ou obtenus à partir de matières premières renouvelables issues de la biomasse.

Conformément à la formule (I), le composé selon l'invention comprend un groupe PAr désignant un groupe polyaromatique comprenant au moins deux cycles hydrocarbures aromatiques condensés.

On entend au sens de la présente invention par « cycle hydrocarbure aromatique » un cycle aromatique dont le squelette est constitué d'atomes de carbone. Autrement dit, il n'y a pas d'hétéroatome dans le squelette du cycle.

On entend au sens de la présente invention par « au moins deux cycles hydrocarbures aromatiques condensés » deux ou plusieurs cycles hydrocarbures aromatiques ayant en commun au moins deux atomes de carbone successifs.

Lesdits au moins deux cycles hydrocarbures aromatiques condensés peuvent être ortho-condensés ou ortho- et péri-condensés.

Avantageusement, le groupe polyaromatique comprenant au moins deux cycles hydrocarbures aromatiques condensés est choisi parmi un groupe formé de deux cycles aromatiques condensés selon un arrangement rectiligne et un groupe formé de trois cycles aromatiques condensés selon un arrangement rectiligne, de préférence un groupe formé de deux cycles aromatiques condensés selon un arrangement rectiligne. Ces structures s'apparentent aux composés du naphtalène et de l'anthracène.

Conformément à la formule (I), le groupe polyaromatique comprenant au moins deux cycles hydrocarbures aromatiques condensés est au moins substitué par la fonction oxyde de nitrile et le groupe Sp. Autrement dit, le premier cycle desdits au moins deux cycles hydrocarbures aromatiques est au moins substitué par la fonction oxyde de nitrile, ledit premier cycle ou le deuxième cycle desdits au moins deux cycles hydrocarbures aromatiques étant au moins substitué par le groupe Sp, ledit groupe Sp permettant de relier la fonction imidazolidinone à l'un au moins desdits au moins deux cycles hydrocarbures aromatiques.

Avantageusement, chacun des cycles hydrocarbures aromatiques condensés est éventuellement substitué par une ou plusieurs chaînes carbonées, identiques ou différentes, aliphatiques ou aromatiques, linéaires, ramifiées ou cycliques, éventuellement substituées ou interrompues par un ou plusieurs hétéroatomes, et inertes vis-à-vis de l'une ou l'autre des fonctions que sont la fonction imidazolidinone et la fonction oxyde de nitrile.

On entend au sens de la présente invention par « chaîne carbonée inerte vis-à-vis de l'une ou l'autre des fonctions que sont la fonction imidazolidinone et la fonction oxyde de nitrile » une chaîne carbonée qui ne réagit pas avec l'une ou l'autre de ces fonctions. Ainsi, ladite chaîne carbonée inerte vis-à-vis de l'une ou l'autre de ces fonctions est par exemple une chaîne carbonée qui ne présente pas de fonctions alcényle ou alcynyle, susceptibles de réagir avec la fonction oxyde de nitrile.

De manière préférée, lesdites chaînes carbonées sont saturées.

De préférence, chacun des cycles hydrocarbures aromatiques condensés est éventuellement substitué par une ou plusieurs chaînes carbonées, identiques ou différentes, saturées, plus préférentiellement un ou plusieurs groupes alkyles, identiques ou différents, préférentiellement un ou plusieurs groupes alkyles, identiques ou différents, en C₁-C₁₂, plus préférentiellement en C₁-C₆, encore plus préférentiellement en C₁-C₄, ou un groupe choisi parmi -OR', -NHR',-SR', R' étant un groupe alkyle, préférentiellement un groupe alkyle en C₁-C₁₂, plus préférentiellement en C₁-C₆, encore plus préférentiellement en C₁-C₄.

Encore plus préférentiellement, chacun des cycles aromatiques condensés est éventuellement substitué par un ou plusieurs groupes, identiques ou différents, méthyle, éthyle ou un ou plusieurs groupes OCH₃.

Le groupe Sp est de préférence une chaîne hydrocarbonée en C₁-C₂₄, linéaire, ramifiée, ou cyclique, pouvant contenir un ou plusieurs radicaux aromatiques, et/ou un ou plusieurs hétéroatomes. Ladite chaîne peut éventuellement être substituée, pour autant que les substituants ne réagissent pas avec l'une ou l'autre des fonctions que sont la fonction imidazolidinone et la fonction oxyde de nitrile.

On entend au sens de la présente invention par « chaîne hydrocarbonée » une chaîne comprenant un ou plusieurs atomes de carbone et un ou plusieurs atomes d'hydrogène.

On entend au sens de la présente invention par « chaîne hydrocarbonée pouvant contenir un ou plusieurs hétéroatomes » le fait que la chaîne peut être substituée ou interrompue par un ou plusieurs hétéroatomes. Lorsque la chaîne est interrompue par un hétéroatome, celui-ci peut être en bout de chaîne ou en milieu de chaîne.

Avantageusement, le groupe Sp est une chaîne alkylène linéaire ou ramifiée en C₁-C₂₄, préférentiellement en C₁-C₁₀, plus préférentiellement en C₁-C₆, éventuellement interrompue par un ou plusieurs atomes d'azote, de soufre ou d'oxygène.

De préférence, le groupe Sp contient un motif choisi parmi-(CH₂)_{y1}-, -[NH-(CH₂)_{y2}]ₓ₁- et -[O-(CH₂)_{y3}]ₓ₂-, [-S-(CH₂)_{y2}]ₓ₁, y₁, y₂ et y₃ représentant, indépendamment les uns des autres, un nombre entier allant de 1 à 6, et x₁ et x₂ représentant, indépendamment l'un de l'autre, un nombre entier allant de 1 à 4.

De préférence, le composé de formule (I) selon l'invention est de formule (II) suivante : 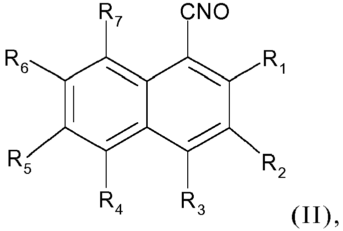 dans laquelle :
- un groupement choisi parmi R₁ à R₇ désigne le groupe de formule (III) suivante : 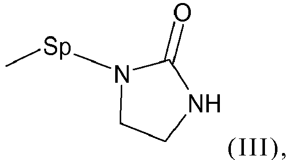
dans laquelle Sp est tel que défini précédemment,
les six autres groupements, identiques ou différents, représentant, indépendamment les uns des autres, un atome d'hydrogène ou une chaîne carbonée, aliphatique ou aromatique, linéaire, ramifiée ou cyclique, éventuellement substituée ou interrompue par un ou plusieurs hétéroatomes.

Avantageusement, ladite chaîne carbonée est inerte vis-à-vis de l'une ou l'autre des fonctions que sont la fonction imidazolidinone et la fonction oxyde de nitrile.

De manière préférée, ladite chaîne carbonée est saturée.

Ladite chaîne carbonée peut être un groupe alkyle en C₁-C₁₂, plus préférentiellement en C₁-C₆, encore plus préférentiellement en C₁-C₄, ou un groupe choisi parmi -OR', -NHR', -SR', R' étant un groupe alkyle, préférentiellement un groupe alkyle en C₁-C₁₂, plus préférentiellement en C₁-C₆, encore plus préférentiellement en C₁-C₄.

De manière préférée, R₁ ou R₇ désigne le groupe de formule (III) .

Selon un mode de réalisation particulier, R₁ désigne le groupe de formule (III) et R₂ à R₇ représentent un atome d'hydrogène.

Selon un autre mode de réalisation particulier, R₇ désigne le groupe de formule (III) et R₁ à R₆ représentent un atome d'hydrogène.

De préférence, le groupe Sp désigne le groupe -O-CH₂-CH₂-.

Selon un mode de réalisation particulier, le composé de formule (I) selon l'invention est choisi parmi un composé de formule (IV) suivante : 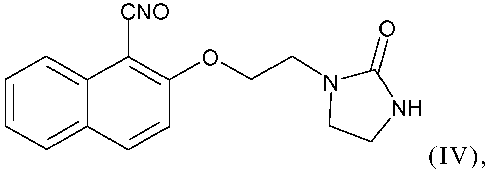 et un composé de formule (V) suivante : 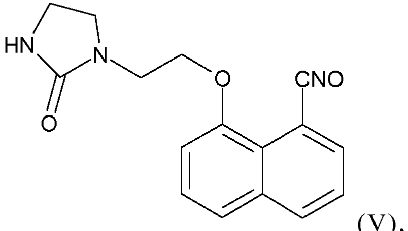 de préférence le composé de formule (IV).

L'invention a également pour objet un procédé de synthèse du composé de formule (I) selon l'invention, comprenant les étapes successives suivantes :
(b1) la réaction d'un composé de formule (VI) suivante : 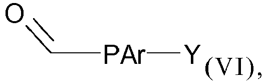 dans laquelle PAr est tel que défini précédemment et Y représente un groupe nucléophile,
   avec un composé de formule (VII) suivante : 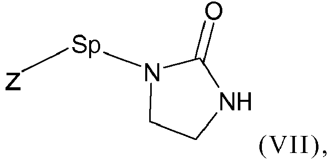 dans laquelle Sp est tel que défini précédemment, et Z représente un groupe nucléofuge ;
   en présence d'au moins un solvant polaire S₁, d'au moins une base, à une température T₁ allant de 70 à 150°C,
   pour former un composé de formule (VIII) suivante : 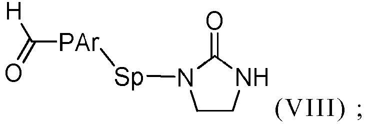
(b2) la réaction dudit composé de formule (VIII) avec une solution aqueuse d'hydroxylamine à une température T₂ allant de 30 à 70°C, pour obtenir un composé oxime de formule (IX) suivante : 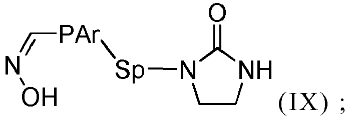
(c) une étape de récupération dudit composé oxime de formule (IX) ;
(d) une étape d'oxydation du composé oxime de formule (IX) avec un agent oxydant, en présence d'au moins un solvant organique S₂.

On entend au sens de la présente invention par « solvant polaire » un solvant présentant une constante diélectrique supérieure à 2,2.

On entend au sens de la présente invention « par groupe nucléofuge » un groupe partant qui emporte son doublet de liaison.

On entend au sens de la présente invention « par groupe nucléophile » un composé comprenant au moins un atome porteur d'un doublet libre ou d'un atome chargé négativement.

Comme expliqué précédemment, le procédé de synthèse du composé de formule (I) selon l'invention comprend notamment les étapes successives (b1) et (b2).

Selon un mode de réalisation particulier, les deux étapes (b1) et (b2) sont séparées par une étape d'isolation et de purification du composé de formule (VIII).

Selon un autre mode de réalisation, les deux étapes (b1) et (b2) sont réalisées selon une synthèse monotope, c'est-à-dire que les étapes (b1) et (b2) sont « one pot » (procédé de synthèse monotope en deux étapes), soit sans isolation du composé de formule (VIII) intermédiaire.

Le procédé selon l'invention comprend une étape (b1) de réaction d'un composé de formule (VI), telle que mentionnée ci-dessus, porteur d'un groupe Y, avec un composé de formule (VII), telle que mentionnée ci-dessus, porteur d'un groupe Z.

De manière préférée, le groupe Y est choisi parmi les fonctions hydroxyle, thiol et amine primaire ou secondaire.

Le groupe Z peut être choisi parmi le chlore, le brome, l'iode, le groupe mésylate, le groupe tosylate, le groupe acétate et le groupe trifluorométhylsulfonate.

Ladite étape (b1) du procédé selon l'invention est réalisée en présence d'au moins un solvant polaire S₁, et d'au moins une base, à une température T₁ allant de 70 à 150°C.

Selon un mode de réalisation particulier de l'invention, le solvant polaire S₁ est un solvant polaire miscible dans l'eau, préférentiellement un solvant protique.

Le diméthylformamide (DMF), le diméthylsulfoxyde (DMSO), la 1,3-diméthyl-2-imidazolidinone (DMI), la 1,3-diméthyl-3,4,5,6-tetrahydro-2(1H)-pyrimidinone (DMPU), l'isopropanol, l'acétonitrile, l'éthanol, le n-butanol et le n-propanol sont des exemples de solvants S₁ utilisables dans le procédé selon l'invention.

De préférence, le solvant protique est alcoolique.

Avantageusement, le composé de formule (VI) représente de 5 à 40% en poids, de préférence de 10 à 30% en poids, par rapport au poids du solvant.

La base peut être choisie parmi les alcoolates alcalins, les carbonates alcalins, les carbonates alcalino-terreux, les hydroxydes alcalins, les hydroxydes alcalino-terreux et leurs mélanges.

Avantageusement, il est possible d'ajouter :
- un ou plusieurs catalyseurs choisis parmi un catalyseur de type sel d'argent (I), un catalyseur de transfert de phase de type ammonium quaternaire, et leurs mélanges ;
- un ou plusieurs liquides ioniques.

Préférentiellement, la base est choisie parmi le méthanolate de sodium, le carbonate de potassium et la soude, plus préférentiellement le carbonate de potassium.

Selon un mode de réalisation particulier de l'invention, la quantité molaire de base est de 1,5 à 8 équivalents molaires, de préférence de 2 à 6 équivalents molaires, par rapport à la quantité molaire de composé de formule (VI).

Comme expliqué précédemment, l'étape (b1) du procédé selon l'invention est réalisée à une température T₁ allant de 70 à 150°C.

De préférence, la température T₁ est une température allant de 70 à 120°C, plus préférentiellement de 80 à 110°C.

Comme expliqué précédemment, l'étape (b1) du procédé selon l'invention est suivie de l'étape (b2) de l'ajout dans le milieu réactionnel contenant le composé de formule (VIII) d'une solution aqueuse d'hydroxylamine à une température T₂ allant de 30 à 70°C.

Préférentiellement, l'ajout de la solution aqueuse d'hydroxylamine est réalisé lorsque la conversion du composé de formule (VI) est d'au moins 70% en poids.

Avantageusement, la température T₂ varie de 40 à 60°C.

Le procédé selon l'invention comprend également une étape (c) de récupération, telle que mentionnée ci-avant, du composé oxime de formule (IX).

De manière préférée, le composé oxime de formule (IX) est récupéré par précipitation à l'eau, suivie éventuellement d'un lavage à l'eau.

Le procédé selon l'invention comprend également une étape (d) d'oxydation du composé oxime de formule (IX) avec un agent oxydant, en présence d'au moins un solvant organique S₂.

De manière préférée, ledit agent oxydant est choisi parmi l'hypochlorite de sodium, le N-bromosuccinimide en présence d'une base, le N-chlorosuccinimide en présence d'une base, et l'eau oxygénée en présence d'un catalyseur, préférentiellement l'hypochlorite de sodium.

Avantageusement, la quantité d'agent oxydant est de 1 à 5 équivalents molaires, préférentiellement de 1 à 2 équivalents molaires, par rapport à la quantité molaire de composé oxime de formule (IX).

Préférentiellement, le solvant organique S₂ est un solvant organique choisi parmi les solvants chlorés et de type ester, éther et alcool, plus préférentiellement choisi parmi le dichlorométhane, l'acétate d'éthyle, l'acétate de butyle, l'éther diéthylique, l'isopropanol et l'éthanol, encore plus préférentiellement choisi parmi l'acétate d'éthyle et l'acétate de butyle.

De préférence, le composé oxime de formule (IX) représente de 1 à 30% en poids, de préférence de 1 à 20% en poids, par rapport au poids total de l'ensemble comprenant ledit composé oxime de formule (IX), ledit solvant organique S₂ et ledit agent oxydant.

Préférentiellement, le procédé selon l'invention comprend, après l'étape (d), une étape (e) de récupération du composé de formule (I).

Selon un mode de réalisation particulier de l'invention, le procédé selon l'invention comprend une étape (a2) de fabrication du composé de formule (VII), préalable à l'étape (b1), par mise en réaction d'un composé de formule (X) suivante : 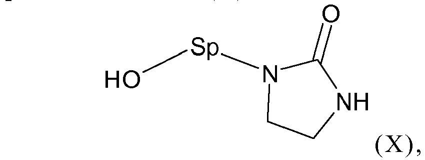 dans laquelle Sp est tel que défini précédemment,
avec un agent permettant la formation du groupe nucléofuge Z.

De manière préférée, ledit agent est le chlorure de thionyle.

De préférence, l'étape (a2) est réalisée en l'absence ou en présence d'au moins un solvant S₄, préférentiellement un solvant chloré, plus préférentiellement le dichlorométhane.

Avantageusement, l'étape (a2) est immédiatement suivie d'une étape (a3) de récupération du composé de formule (VII), préférentiellement par purification au toluène, plus préférentiellement par cristallisation du composé de formule (VII) dans le toluène.

Comme expliqué précédemment, le composé de formule (I) peut être intégré à des compositions de caoutchouc, utilisables notamment pour la fabrication de pneumatiques. Ces compositions de caoutchouc peuvent être à base d'au moins un élastomère diénique, d'une charge renforçante et d'un agent de réticulation chimique.

Dans la suite du texte, on entend par "composition à base de" une composition comportant le mélange et/ou le produit de réaction des différents constituants utilisés, certains de ces constituants de base étant susceptibles de, ou destinés à, réagir entre eux, au moins en partie, lors des différentes phases de fabrication de la composition, en particulier au cours de sa réticulation ou vulcanisation.

Par élastomère (ou indistinctement caoutchouc) diénique, qu'il soit naturel ou synthétique, doit être compris de manière connue un élastomère constitué au moins en partie (i.e ; un homopolymère ou un copolymère) d'unités monomères diènes (monomères porteurs de deux doubles liaisons carbone-carbone, conjuguées ou non).

Le composé de formule (I) peut être utilisé pour greffer un ou plusieurs élastomères diéniques présent(s) dans la composition de caoutchouc. L'élastomère diénique peut être greffé par le composé de formule (I) préalablement à son introduction dans la composition de caoutchouc, ou bien peut être greffé par réaction avec le composé de formule (I) lors de la fabrication de la composition.

La composition de caoutchouc pouvant être comprise dans le pneumatique peut donc contenir un seul élastomère diénique greffé par le composé de formule (I) (soit greffé préalablement à son introduction dans la composition, soit greffé par réaction avec le composé de formule (I) pendant la fabrication de la composition), ou un mélange de plusieurs élastomères diéniques tous greffés, ou dont certains sont greffés et les autres pas.

Le ou les élastomères diéniques greffés peuvent être utilisés en association avec tout type d'élastomère synthétique autre que diénique, voire avec des polymères autres que des élastomères.

La composition de caoutchouc peut également comprendre une charge renforçante.

La charge renforçante peut être de tout type de charge dite renforçante, connue pour ses capacités à renforcer une composition de caoutchouc utilisable pour la fabrication de pneumatiques, par exemple une charge organique telle que du noir de carbone, une charge inorganique renforçante telle que de la silice à laquelle est associé de manière connue un agent de couplage, ou encore un mélange de ces deux types de charge.

Une telle charge renforçante consiste typiquement en des nanoparticules dont la taille moyenne (en masse) est inférieure au micromètre, généralement inférieure à 500 nm, le plus souvent comprise entre 20 et 200 nm, en particulier et plus préférentiellement comprise entre 20 et 150 nm.

Un autre composant de la composition de caoutchouc, telle que mentionnée auparavant, est l'agent de réticulation chimique.

L'agent de réticulation permet la formation de liaisons covalentes entre les chaînes élastomères, ce qui leur confère des propriétés élastiques. L'agent de réticulation peut être à base soit de soufre, soit de donneurs de soufre et/ou de peroxyde et/ou de bismaléimides, de préférence à base de soufre.

La composition de caoutchouc telle que mentionnée ci-dessus peut comporter également tout ou partie des additifs usuels habituellement utilisés dans les compositions d'élastomères destinées à la fabrication de pneumatiques, en particulier de bandes de roulement, comme par exemple des plastifiants, des pigments, des agents de protection comme des antioxydants, des charges non renforçantes.

La présente invention est en outre illustrée par les exemples non limitatifs suivants.

### EXEMPLES

### Molécules

L'analyse structurale ainsi que la détermination des puretés molaires des molécules de synthèses sont réalisées par une analyse RMN. Les spectres sont acquis sur un spectromètre Avance 3 400 MHz BRUKER équipé d'une sonde "large bande" BBFO-zgrad 5 mm. L'expérience RMN ¹H quantitative, utilise une séquence simple impulsion 30° et un délai de répétition de 3 secondes entre chacune des 64 acquisitions. Les échantillons sont solubilisés dans un solvant deutéré, le diméthylsulfoxide deutéré (DMSO) sauf indication contraire. Le solvant deutéré est également utilisé pour le signal de lock. Par exemple, la calibration est réalisée sur le signal des protons du DMSO deutéré à 2.44 ppm par rapport à une référence TMS à 0ppm. Le spectre RMN ¹H couplé aux expériences 2D HSQC ¹H/¹³C et HMBC ¹H/¹³C permettent la détermination structurale des molécules (cf tableaux d'attributions). Les quantifications molaires sont réalisées à partir du spectre RMN 1D ¹H quantitatif.

L'analyse par spectrométrie de masse est réalisée en injection directe par un mode d'ionisation en électrospray (ID/ESI). Les analyses ont été réalisées sur un spectromètre HCT Bruker (débit 600 µL/min, pression du gaz nébuliseur 10 psi, débit du gaz nébuliseur 4 L/min).

### Molécule greffée sur SBR ou IR

La détermination du taux molaire de l'oxyde de 2-[2-(2-oxoimidazolidin-1-yl)éthoxy]-1-naphtonitrile greffé sur SBR (caoutchouc styrène-butadiène) ou IR (caoutchouc isoprène) est réalisée par une analyse RMN. Les spectres sont acquis sur un spectromètre 500 MHz BRUKER équipé d'une «CryoSonde BBFO-zgrad-5 mm». L'expérience RMN ¹H quantitative, utilise une séquence simple impulsion 30° et un délai de répétition de 5 secondes entre chaque acquisition. Les échantillons sont solubilisés dans le chloroforme deutéré (CDCl₃) dans le but d'obtenir un signal de «lock». Des expériences RMN 2D ont permis de vérifier la nature du motif greffé grâce aux déplacements chimiques des atomes de carbone et de proton.

### Essais de traction

Ces essais de traction permettent de déterminer les contraintes d'élasticité et les propriétés à la rupture. Sauf indication différente, ils sont effectués conformément à la norme française NF T 46-002 de septembre 1988.

Les contraintes à la rupture (en MPa) et les allongements à la rupture (en base 100) sont mesurés à 23°C ± 2°C et à 100°C ± 2°C, selon la norme NF T 46-002.

### Propriétés dynamiques

Les propriétés dynamiques ΔG* et tan(δ)max sont mesurées sur un viscoanalyseur (Metravib VA4000), selon la norme ASTM D 5992-96. On enregistre la réponse d'un échantillon de composition vulcanisée (éprouvette cylindrique de 4 mm d'épaisseur et de 400 mm² de section), soumis à une sollicitation sinusoïdale en cisaillement simple alterné, à la fréquence de 10Hz, dans les conditions normales de température (23°C) selon la norme ASTM D 1349-99, ou selon les cas à une température différente (100°C). On effectue un balayage en amplitude de déformation de 0,1% à 100% (cycle aller), puis de 100% à 0,1% (cycle retour). Les résultats exploités sont le module complexe de cisaillement dynamique (G*) et le facteur de perte tan(δ). Pour le cycle retour, on indique la valeur maximale de tan(δ) observée, noté tan(δ)max.

### I. Synthèse de l'oxyde de 2-[2-(2-oxoimidazolidin-1-yl)éthoxy]-1-naphtonitrile

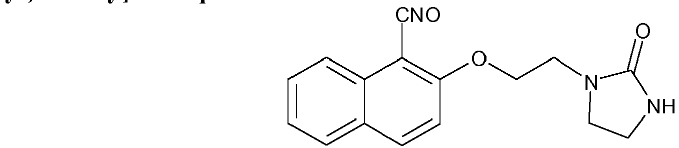

L'oxyde de 2-[2-(2-oxoimidazolidin-1-yl)éthoxy]-1-naphtonitrile est synthétisé en six étapes, nommées étape a1, étape a2, étape b1, étape b2, étape c et étape d.

L'étape a1 est réalisée selon le protocole 1, l'étape a2 selon le protocole 2, l'étape b1 selon le protocole 3. Les étapes b2 et c sont réalisées selon le protocole 4. L'étape d est réalisée selon le protocole 5.

### Etape a1 : préparation du 2-hydroxy-1-naphthaldéhyde

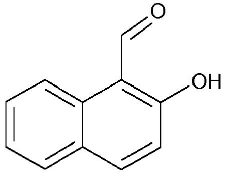

### Protocole 1 :

Ce composé peut être obtenu à partir du napthol par une réaction de formylation selon le protocole dit de Reimer-Tiemann décrit dans Organic Syntheses, 22, 63-4; 1942 par Russell, Alfred et Lockhart, Luther B. ou dans Organic Reactions (Hoboken, NJ, United States), 28, 1982 par Wynberg, Hans et Meijer, Egbert W., ou selon la procédure décrite par Casiraghi, Giovanni et al. dans Journal of the Chemical Society, Perkin Transactions 1: Organic and Bio-Organic Chemistry (1972-1999), (9), 1862-5, 1980 ou encore par une réaction de Vilsmeier décrite par Jones, Gurnos et Stanforth, Stephen P. dans Organic Reactions (Hoboken, NJ, United States), 49, 1997.

Le 2-hydroxy-1-napthaldéhyde est commercial. Il peut par exemple être obtenu chez Aldrich (CAS 708-06-5).

### Etape a2 : préparation de la 1-(2-chloroéthyl)imidazolidin-2-one

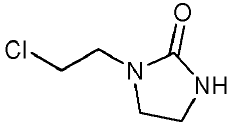

### Protocole 2 :

Ce composé peut être obtenu selon un protocole décrit dans la demande de brevet WO 2012/007684.

### Etape b1 : préparation du 2-(2-(2-oxoimidazolidin-1-yl)éthoxy)-1-naphthaldéhyde

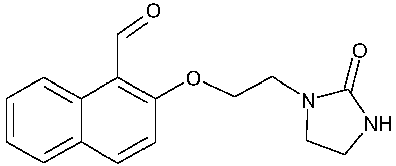

### Protocole 3 :

Un mélange de 2-hydroxy-1-naphthaldéhyde (20,0 g, 0,116 mol), de carbonate de potassium (24,08 g, 0,174 mol), et de 1-(2-chloroéthyl)imidazolidin-2-one (25,9 g, 0,174 mol) dans le DMF (20 mL) est chauffé à 75°C (T_{bain}) pendant 3,0-3,5 heures. Ensuite, une seconde portion de 1-(2-chloroéthyl)imidazolidin-2-one (17,26 g, 0,116 mol) est ajoutée et le milieu réactionnel est agité pendant 9 à 10 heures à 75°C (T_{bain}). Après un retour à 40-45°C, le milieu réactionnel est versé dans une solution d'hydroxyde de sodium (10g, 0,25 mol) dans l'eau (700 mL). Le mélange est agité pendant 10 à 15 minutes. Le précipité est filtré et lavé sur le filtre par l'eau (3 fois 400 mL).

Un solide gris (31,51g, rendement massique de 95 %) est obtenu.

La pureté molaire est supérieure à 85 % (RMN ¹H).

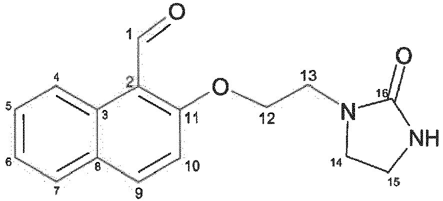

| N° | δ ¹H (ppm) | δ ¹³C (ppm) |
|---|---|---|
| 1 | 10.86 | 191.6 |
| 2 | / | 116.7 |
| 3 | / | 131.5 |
| 4 | 9.19 | 124.9 |
| 5 | 7.56 | 130 |
| 6 | 7.37 | 124.9 |
| 7 | 7.71 | 128.3 |
| 8 | / | 128.6 |
| 9 | 7.99 | 137.7 |
| 10 | 7.2 | 113.1 |
| 11 | / | 162.9 |
| 12 | 4.31 | 68.7 |
| 13 | 3.63 | 13.4 |
| 14 | 3.59 | 16.8 |
| 15 | 3.72 | 38.3 |
| 16 | / | 162.6 |

Solvant : CDCl₃

### Etapes b2 et c : préparation de l'oxime de 2-(2-(2-oxoimidazolidin-1-yl)éthoxy)-1-naphthaldéhyde

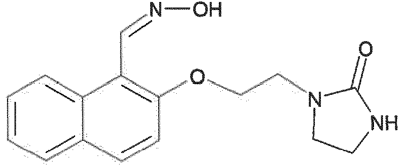

Le produit isolé à l'issue du protocole 3 est utilisé dans le protocole 4 suivant.

### Protocole 4 :

A une solution de 2-(2-(2-oxoimidazolidin-1-yl)éthoxy)-1-naphthaldéhyde (6,3 g, 22,2 mmol) dans l'éthanol (30 mL) à 45°C (T_{bain}), est ajoutée une solution d'hydroxylamine (2,05 g, 31,0 mmol, 50 % dans l'eau, Aldrich) dans l'éthanol (5 mL). Le milieu réactionnel est agité pendant 4 heures à 55 °C (T_{bain}). Après un retour à 40°C, de l'acétate d'éthyle (30 mL) est ajouté goutte à goutte pendant 15 minutes. Le précipité est filtré, lavé sur le filtre par un mélange éthanol/acétate d'éthyle.

Un solide blanc (4,00g, rendement massique de 60 %) est obtenu.

La pureté molaire estimée par RMN ¹H est supérieure à 95 %.

CCM : Rf = 0,10 (SiO₂ ; EtOAc) ; révélation par UV et I₂.

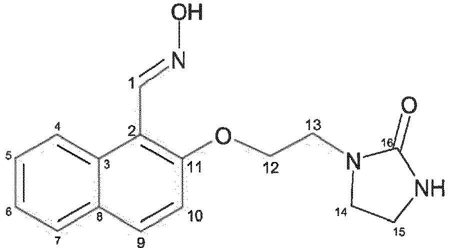

| N° | δ ¹H (ppm) | δ ¹³C (ppm) |
|---|---|---|
| 1 | 8.75 | 147.5 |
| 2 | / | 115.9 |
| 3 | / | 133 |
| 4 | 8.78 | 127 |
| 5 | 7.46 | 128.5 |
| 6 | 7.35 | 125.2 |
| 7 | 7.88 | 132.8 |
| 8 | / | 130.9 |
| 9 | 7.79 | 129.4 |
| 10 | 7.36 | 115.2 |
| 11 | / | 157.1 |
| 12 | 4.28 | 69.3 |
| 13 | 3.59 | 44.4 |
| 14 | 3.64 | 47.6 |
| 15 | 3.40 | 39.3 |
| 16 | / | 165.3 |

Solvant : MeOH

### Etape d : préparation de l'oxyde de 2-[2-(2-oxoimidazolidin-1-yl)éthoxy]-1-naphtonitrile

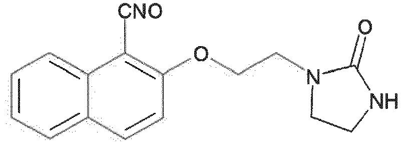

### Protocole 5 :

A une solution d'oxime de 2-(2-(2-oxoimidazolidin-1-yl)éthoxy)-1-naphthaldéhyde (7,5g, 25,06 mmol) dans le dichlorométhane (300 mL) refroidi à 4°C (T_{bain} = 0°C), est ajoutée, goutte à goutte, une solution aqueuse de NaOCl (78 % g Cl/L, 35 mL) pendant 15 minutes. Le milieu réactionnel est agité pendant 60 à 70 minutes à une température de 4-5°C. La phase organique est séparée. La phase aqueuse est extraite par le dichlorométhane (25 mL). Les solutions organiques réunies sont lavées par l'eau (2 fois par 25 mL). Le solvant est évaporé sous pression réduite jusqu'à 40-50 mL. De l'éther de pétrole 40/60 (60 mL) est ajouté pour la cristallisation. Le précipité obtenu est filtré et lavé par l'éther de pétrole 40/60 (2 fois par 30 mL).

Un solide blanc (6,46 g, rendement massique de 87 %) est obtenu.

La pureté molaire estimée par RMN ¹H est supérieure à 99 %.

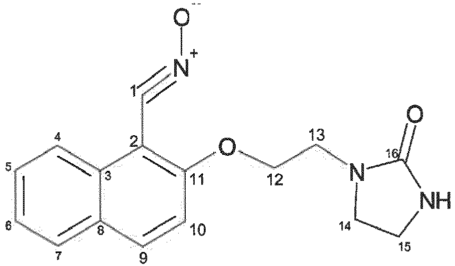

| N° | δ ¹H (ppm) | δ ¹³C (ppm) | N° | δ ¹H (ppm) | δ ¹³C (ppm) |
|---|---|---|---|---|---|
| 1 | / | / | 9 | 7.89 | 133 |
| 2 | / | 96.7 | 10 | 7.18 | 112.7 |
| 3 | / | 134.1 | 11 | / | 160.5 |
| 4 | 7.92 | 124 | 12 | 4.31 | 69.4 |
| 5 | 7.56 | 128.9 | 13 | 3.64 | 43.4 |
| 6 | 7.39 | 125.2 | 14 | 3.76 | 47.3 |
| 7 | 7.77 | 128.6 | 15 | 3.42 | 38.5 |
| 8 | / | 128.5 | 16 | / | 162.5 |

Solvant : CDCl₃

### II. Fabrication d'un SBR greffé par l'oxyde de 2-[2-(2-oxoimidazolidin-1-yl)éthoxy]-1-naphtonitrile

L'oxyde de 2-[2-(2-oxoimidazolidin-1-yl)éthoxy]-1-naphtonitrile obtenu précédemment (à l'issue du protocole 5) est utilisé.

On incorpore de l'oxyde de 2-[2-(2-oxoimidazolidin-1-yl)éthoxy]-1-naphtonitrile (0,88g ; 2,97 mmol) à 20g de SBR (contenant 26,5% en poids de styrène et 25% en poids d'unité butadiène-1,2, de Mn=150000 g/mol et Ip=1,84) sur outil à cylindres (mélangeur externe à 23°C). Le mélange est homogénéisé en 15 passes portefeuille. Cette phase de mélangeage est suivie d'un traitement thermique à 120°C pendant 10 minutes sous presse à 10 bars de pression.

L'analyse par RMN ¹H a permis de démontrer un taux molaire de greffage de 0,81% avec un rendement molaire de greffage de 80%.

| N° | δ ¹H (ppm) | δ ¹³C (ppm) |
|---|---|---|
| 1 | / | - |
| 2 | / | - |
| 3 | / | - |
| 4 | 7,70 | 129,5 |
| 5 | 7,61 | 126,7 |
| 6 | 7,77 | 123,7 |
| 7 | 7,29 | 125,9 |
| 8 | / | - |
| 9 | 7,21 | 123,0 |
| 10 | 7,10 | 112,3 |
| 11 | / | - |
| 12 | 4,11 | 66,5 |
| 13 | 3,33 | 44,6 |
| 14 | 3,34 + 3,49 (H non eq*) | 42,0 |
| 15 | 3,18 | 37,2 |
| 16 | / | - |

| | | |
|---|---|---|
| (*) ¹H non équivalents : protons portés par le même atome de carbone n'ayant pas le même déplacement chimique ¹H. | | |

### III. Fabrication d'un IR greffé par l'oxyde de 2-[2-(2-oxoimidazolidin-1-yl)éthoxy]-1-naphtonitrile

L'oxyde de 2-[2-(2-oxoimidazolidin-1-yl)éthoxy]-1-naphtonitrile obtenu précédemment (à l'issue du protocole 5) est utilisé.

On incorpore de l'oxyde de 2-[2-(2-oxoimidazolidin-1-yl)éthoxy]-1-naphtonitrile (0,26g ; 0,88mmol) à 20g d'IR (contenant 98% en poids d'unité isoprène-1,4 cis et de Mn=375000 g/mol et Ip=3,6) sur outil à cylindres (mélangeur externe à 23°C). Le mélange est homogénéisé en 15 passes portefeuille. Cette phase de mélangeage est suivie d'un traitement thermique à 120°C pendant 10 minutes sous presse à 10 bars de pression.

L'analyse par RMN ¹H a permis de démontrer un taux molaire de greffage de 0,26% avec un rendement molaire de greffage de 90%.

| N° | δ ¹H (ppm) | δ ¹³C (ppm) |
|---|---|---|
| 1 | / | - |
| 2 | / | - |
| 3 | / | - |
| 4 | 7,83 | 131,2 |
| 5 | 7,72 | 128,1 |
| 6 | 7,86 | 124,3 |
| 7 | 7,42 | 127,5 |
| 8 | / | - |
| 9 | 7,30 | 124,2 |
| 10 | 7,20 | 113,3 |
| 11 | / | - |
| 12 | 4,23 | 68,3 |
| 13 | 3,53 | 46,5 |
| 14 | 3,56 | 43,6 |
| 15 | 3,33 | 38,4 |
| 16 | / | - |

### IV. Compositions de caoutchouc

### 1) Préparation des compositions de caoutchouc

Quatre compositions de caoutchouc sont préparées.

Des compositions de caoutchouc comprenant le SBR ou l'IR greffé par l'oxyde de 2-[2-(2-oxoimidazolidin-1-yl)éthoxy]-1-naphtonitrile (composé de formule (IV) selon l'invention, appelé dans la suite composé A) sont préparées. Des compositions de caoutchouc comprenant le SBR ou l'IR greffé par l'oxyde de 2,4,6-triméthyl-3-[2-(2-oxoimidazolidin-1-yl)éthoxy]benzonitrile (appelé dans la suite composé B ; composé comparatif) sont également préparées.

On procède pour les essais qui suivent de la manière suivante selon deux cas :
- Cas A : on introduit dans un mélangeur interne Polylab de 85cm³, rempli à 70% et dont la température initiale de cuve est d'environ 110°C, le ou les élastomères diéniques greffés comme cela est décrit au point II ou au point III.
- Cas B : on introduit dans un mélangeur interne Polylab de 85cm³, rempli à 70% et dont la température initiale de cuve est d'environ 110°C, le ou les élastomères diéniques non greffés. Pour les mélanges concernant le composé A et le composé B, le composé A ou le composé B est introduit en même temps que l'élastomère diénique et est conduit un travail thermomécanique d'une minute à 120°C.

Ensuite, pour chacune des quatre compositions, on introduit la ou les charges renforçantes éventuelles, l'agent de couplage éventuel puis, après une à deux minutes de malaxage, les divers autres ingrédients à l'exception du système de vulcanisation. On conduit alors un travail thermomécanique (phase non-productive) en une étape (durée totale du malaxage égale à environ 5 minutes), jusqu'à atteindre une température maximale de "tombée" de 160°C. On récupère le mélange ainsi obtenu, on le refroidit puis on ajoute le système de vulcanisation (soufre) sur un mélangeur externe (homo-finisseur) à 25°C, en mélangeant le tout (phase productive) pendant environ 5 à 6 minutes.

Les compositions ainsi obtenues sont ensuite calandrées soit sous la forme de plaques (épaisseur de 2 à 3 mm) ou de feuilles fines de caoutchouc pour la mesure de leurs propriétés physiques ou mécaniques, soit sous la forme de profilés utilisables directement, après découpage et/ou assemblage aux dimensions souhaitées, par exemple comme produits semi-finis pour pneumatiques, en particulier comme bandes de roulement de pneumatiques.

### a) Compositions de caoutchouc SBR

Les compositions de caoutchouc concernant le SBR sont données dans le tableau 1. La composition de caoutchouc comprenant le SBR greffé par le composé A selon l'invention est appelée composition 1. La composition de caoutchouc comprenant le SBR greffé par le composé B comparatif est appelée composition 2. Les teneurs sont exprimées en pce. Le taux molaire de composé greffé sur le SBR est de 0,3% aussi bien pour le SBR-g-A que le SBR-g-B. Ainsi, il y a 0,3 mole de composé greffé pour 100 moles de motifs du polymère (moles de motifs monomères constituant le polymère, y compris ceux qui portent le composé greffé).

**Tableau 1**

| Compositions | 1 | 2 |
|---|---|---|
| SBR-g-A (1) | 101,4 | - |
| SBR-g-B (2) | - | 101,3 |
| Silice (3) | 55 | 55 |
| Silane TESPT (4) | 5,5 | 5,5 |
| N234 (5) | 3 | 3 |
| 6PPD (6) | 1,5 | 1,5 |
| TMQ (7) | 1 | 1 |
| Paraffine 6266 (8) | 1 | 1 |
| ZnO (9) | 2,7 | 2,7 |
| Acide stéarique (10) | 2,5 | 2,5 |
| CBS (11) | 1,8 | 1,8 |
| Soufre (12) | 1,5 | 1,5 |

| | | |
|---|---|---|
| (1) SBR greffé par le composé A (2) SBR greffé par le composé B (3) Silice « Zeosil 1165MP » - société Rhodia sous forme de microperles (BET et CTAB d'environ 150-160 m²/g) (4) TESPT (« S169 » - société Degussa) (5) Noir de carbone N234 (6) Antioxydant « Santoflex 6PPD » - société Solutia (7) 2,2,4-triméthyl-1,2-dihydroquinoline - société Flexys (8) Paraffine (9) Oxyde de zinc (grade industriel - Société Umicore) (10) Stéarine (« Pristerene 4031 » - Société Uniquema) (11) CBS : N-cyclohexyl-2-benzothiazyl-sulfénamide (« Santocure CBS » - Société Flexys) | | |

### b) Compositions de caoutchouc IR

Les compositions de caoutchouc concernant l'IR sont données dans le tableau 2. La composition de caoutchouc comprenant l'IR greffé par le composé A selon l'invention est appelée composition 3. La composition de caoutchouc comprenant l'IR greffé par le composé B comparatif est appelée composition 4. La composition de caoutchouc comprenant l'IR greffé par l'oxyde de 2-[2-(2-oxoimidazolidin-1-yl)éthoxy]benzonitrile (appelé dans la suite composé C) est appelée composition 5, préparée selon un protocole identique au cas B si ce n'est qu'on utilise le composé C comparatif. Les deux composés B et C sont décrits dans la demande WO 2016/207263. Les teneurs sont exprimées en pce. Le taux molaire de composé greffé sur le IR est de 0,6% aussi bien pour le IR-g-A que le IR-g-B et le IR-g-C. Ainsi, il y a 0,6 mole de composé greffé pour 100 moles de motifs du polymère (moles de motifs monomères constituant le polymère, y compris ceux qui portent le composé greffé).

**Tableau 2**

| Compositions | 3 | 4 | 5 |
|---|---|---|---|
| IR-g-A (1) | 102,6 | - | - |
| IR-g-B (2) | - | 103,5 | - |
| IR-g-C (3) | | | 102.2 |
| Silice (4) | 55 | 55 | 55 |
| Silane TESPT (5) | 5,5 | 5,5 | 5,5 |
| N234 (6) | 3 | 3 | 3 |
| 6PPD (7) | 1,5 | 1,5 | 1,5 |
| TMQ (8) | 1 | 1 | 1 |
| Paraffine 6266 (9) | 1 | 1 | 1 |
| ZnO (10) | 2,7 | 2,7 | 2,7 |
| Acide stéarique (11) | 2,5 | 2,5 | 2,5 |
| CBS (12) | 1,8 | 1,8 | 1,8 |
| Soufre | 1,5 | 1,5 | 1,5 |

| | | | |
|---|---|---|---|
| (1) IR greffé par le composé A (2) IR greffé par le composé B (3) IR greffé par le composé C (4) Silice « Zeosil 1165MP » - société Rhodia sous forme de microperles (BET et CTAB d'environ 150-160 m²/g) (5) TESPT (« S169 » - société Degussa) (6) Noir de carbone N234 (7) Antioxydant « Santoflex 6PPD » - société Solutia (8) 2,2,4-triméthyl-1,2-dihydroquinoline (9) Paraffine (10) Oxyde de zinc (grade industriel - Société Umicore) (11) Stéarine (« Pristerene 4031 » - Société Uniquema) (12) CBS : N-cyclohexyl-2-benzothiazyl-sulfénamide (« Santocure CBS » - Société Flexys) | | | |

### 2) Essais de caractérisation - Résultats

Lorsque les résultats sont présentés en base 100 par rapport à un témoin, la valeur indiquée pour une composition est le rapport entre la valeur mesurée sur la composition et la valeur mesurée sur la composition témoin.

### a) Compositions de caoutchouc SBR

Les propriétés des compositions 1 et 2 sont comparées. Les résultats sont reportés dans le tableau 3 :

**Tableau 3**

| Compositions | 1 | 2 |
|---|---|---|
| **Propriétés en élongation à 23°C (base 100)** | | |
| Contrainte rupture | 100 | 100 |

| **Propriétés en élongation à 100°C (base 100)** | | |
|---|---|---|
| Contrainte rupture | 106 | 100 |

| **Propriétés dynamiques à 23°C** | | |
|---|---|---|
| G* 10% (MPa) | 3,41 | 3,32 |
| Tan δ max | 0,17 | 0,17 |

| **Propriétés dynamiques à 100°C** | | |
|---|---|---|
| G* 10% (MPa) | 2,18 | 2,05 |
| Tan δ max | 0,11 | 0,12 |

La composition 1 présente à 100°C une meilleure contrainte à la rupture que la composition 2. Par ailleurs, la composition 1 présente une meilleure rigidité que la composition 2, accompagnée d'une conservation des propriétés d'hystérèse.

La composition comprenant le composé selon l'invention possède donc des propriétés améliorées par rapport à la composition comprenant un composé comparatif.

### b) Compositions de caoutchouc IR

Les propriétés des compositions 3 et 4 sont comparées. Les résultats sont reportés dans le tableau 4 :

**Tableau 4**

| Compositions | 3 | 4 |
|---|---|---|
| **Propriétés en élongation à 23°C (base 100)** | | |
| Contrainte rupture | 111 | 100 |

| **Propriétés en élongation à 100°C (base 100)** | | |
|---|---|---|
| Contrainte rupture | 102 | 100 |

| **Propriétés dynamiques à 23°C** | | |
|---|---|---|
| G* 10% (MPa) | 1,56 | 1,55 |
| Tan δ max | 0,11 | 0,10 |

| **Propriétés dynamiques à 100°C** | | |
|---|---|---|
| G* 10% (MPa) | 1,12 | 1,11 |
| Tan δ max | 0,07 | 0,06 |

La composition 3 présente une meilleure contrainte à la rupture que la composition 4, accompagnée d'une conservation des propriétés de rigidité et d'hystérèse, quelle que soit la température.

Les propriétés des compositions 3 et 5 sont comparées. Les résultats sont reportés dans le tableau 5 :

**Tableau 5**

| Compositions | 3 | 5 |
|---|---|---|
| **Propriétés en élongation à 23°C (base 100)** | | |
| Contrainte rupture | 96 | 100 |

| **Propriétés en élongation à 100°C (base 100)** | | |
|---|---|---|
| Contrainte rupture | 148 | 100 |

| **Propriétés dynamiques à 23°C** | | |
|---|---|---|
| G* 10% (MPa) | 104 | 100 |
| Tan δ max | 93 | 100 |

| **Propriétés dynamiques à 100°C** | | |
|---|---|---|
| G* 10% (MPa) | 98 | 100 |
| Tan δ max | 95 | 100 |

La composition 3 présente à 100°C une bien meilleure contrainte à la rupture que la composition 5, accompagnée d'une conservation des propriétés de rigidité et d'hystérèse.

La composition comprenant le composé selon l'invention possède donc des propriétés améliorées par rapport à la composition comprenant un composé comparatif.

## Revendications

1. Composé de formule (I) suivante : 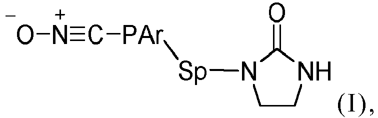 dans laquelle :
- PAr désigne un groupe polyaromatique comprenant au moins deux cycles hydrocarbures aromatiques condensés, chacun desdits cycles aromatiques condensés étant éventuellement substitué par une ou plusieurs chaînes carbonées, identiques ou différentes, indépendantes les unes des autres, aliphatiques ou aromatiques, linéaires, ramifiées ou cycliques, éventuellement substituées ou interrompues par un ou plusieurs hétéroatomes ;
- Sp représente un atome ou un groupe d'atomes.

2. Composé selon la revendication 1, **caractérisé en ce que** le groupe Sp est une chaîne alkylène linéaire ou ramifiée en C₁-C₂₄, préférentiellement en C₁-C₁₀, plus préférentiellement en C₁-C₆, éventuellement interrompue par un ou plusieurs atomes d'azote, de soufre ou d'oxygène.

3. Composé selon la revendication 1 ou 2, **caractérisé en ce qu'**il est de formule (II) suivante : 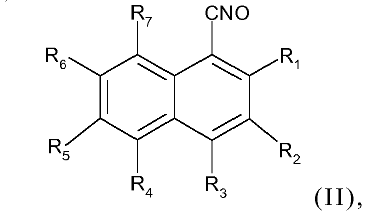 dans laquelle :
- un groupement choisi parmi R₁ à R₇ désigne le groupe de formule (III) suivante : 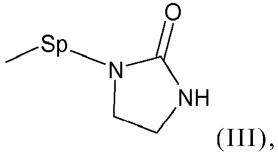
dans laquelle Sp est tel que défini à la revendication 1 ou 2,
les six autres groupements, identiques ou différents, représentant, indépendamment les uns des autres, un atome d'hydrogène ou une chaîne carbonée, aliphatique ou aromatique, linéaire, ramifiée ou cyclique, éventuellement substituée ou interrompue par un ou plusieurs hétéroatomes.

4. Composé selon l'une quelconque des revendications précédentes, **caractérisé en ce qu'**il est choisi parmi un composé de formule (IV) suivante : 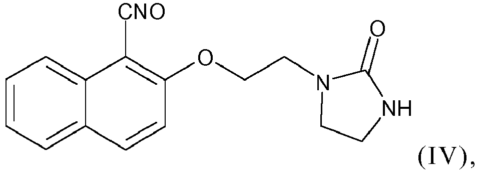 et un composé de formule (V) suivante : 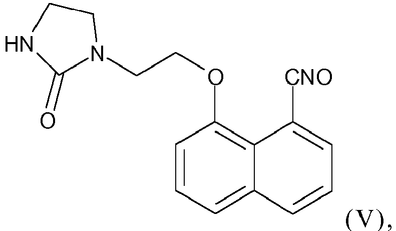 de préférence le composé de formule (IV).

5. Procédé de synthèse du composé de formule (I) tel que défini à l'une quelconque des revendications précédentes, comprenant les étapes successives suivantes :
(b1) la réaction d'un composé de formule (VI) suivante : 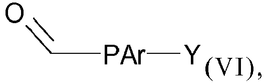 dans laquelle PAr est tel que défini à la revendication 1 et Y représente un groupe nucléophile,
avec un composé de formule (VII) suivante : 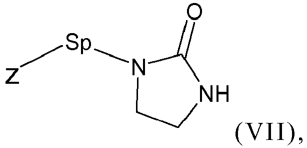 dans laquelle Sp est tel que défini à la revendication 1 ou 2, et Z représente un groupe nucléofuge ;
en présence d'au moins un solvant polaire S₁, d'au moins une base, à une température T₁ allant de 70 à 150°C,
pour former un composé de formule (VIII) suivante : 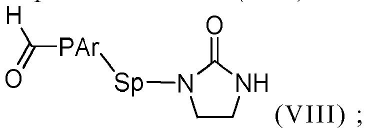
(b2) la réaction dudit composé de formule (VIII) avec une solution aqueuse d'hydroxylamine à une température T₂ allant de 30 à 70°C, pour obtenir un composé oxime de formule (IX) suivante : 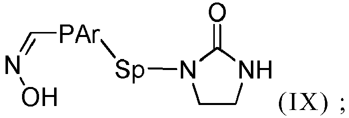
(c) une étape de récupération dudit composé oxime de formule (IX) ;
(d) une étape d'oxydation du composé oxime de formule (IX) avec un agent oxydant, en présence d'au moins un solvant organique S₂.

6. Procédé selon la revendication 5, dans lequel les deux étapes (b1) et (b2) sont séparées par une étape d'isolation et de purification du composé de formule (VIII).

7. Procédé selon la revendication 5, dans lequel les deux étapes (b1) et (b2) sont réalisées selon une synthèse monotope.

8. Procédé selon l'une quelconque des revendications 5 à 7, dans lequel le groupe Y est choisi parmi les fonctions hydroxyle, thiol et amine primaire ou secondaire.

9. Procédé selon l'une quelconque des revendications 5 à 8, dans lequel le groupe Z est choisi parmi le chlore, le brome, l'iode, le groupe mésylate, le groupe tosylate, le groupe acétate et le groupe trifluorométhylsulfonate.

10. Procédé selon l'une quelconque des revendications 5 à 9, dans lequel le solvant polaire S₁ est un solvant polaire miscible dans l'eau, de préférence un solvant protique, plus préférentiellement un solvant protique alcoolique.

11. Procédé selon l'une quelconque des revendications 5 à 10, dans lequel le composé de formule (VI) représente de 5 à 40% en poids, de préférence de 10 à 30% en poids, par rapport au poids du solvant.

12. Procédé selon l'une quelconque des revendications 5 à 11, dans lequel la base est choisie parmi les alcoolates alcalins, les carbonates alcalins, les carbonates alcalino-terreux, les hydroxydes alcalins, les hydroxydes alcalino-terreux et leurs mélanges.

13. Procédé selon l'une quelconque des revendications 5 à 12, dans lequel la base est choisie parmi le méthanolate de sodium, le carbonate de potassium et la soude, préférentiellement le carbonate de potassium.

14. Procédé selon l'une quelconque des revendications 5 à 13, dans lequel la quantité molaire de base est de 1,5 à 8 équivalents molaires, de préférence de 2 à 6 équivalents molaires, par rapport à la quantité molaire de composé de formule (VI).

15. Procédé selon l'une quelconque des revendications 5 à 14, dans lequel l'ajout de la solution aqueuse d'hydroxylamine est réalisé lorsque la conversion du composé de formule (VI) est d'au moins 70% en poids.

16. Procédé selon l'une quelconque des revendications 5 à 15, dans lequel l'agent oxydant est choisi parmi l'hypochlorite de sodium, le N-bromosuccinimide en présence d'une base, le N-chlorosuccinimide en présence d'une base, et l'eau oxygénée en présence d'un catalyseur, préférentiellement l'hypochlorite de sodium.

17. Procédé selon l'une quelconque des revendications 5 à 16, dans lequel le solvant organique S₂ est un solvant organique choisi parmi les solvants chlorés et de type ester, éther et alcool, plus préférentiellement choisi parmi le dichlorométhane, l'acétate d'éthyle, l'acétate de butyle, l'éther diéthylique, l'isopropanol et l'éthanol, encore plus préférentiellement choisi parmi l'acétate d'éthyle et l'acétate de butyle.

18. Procédé selon l'une quelconque des revendications 5 à 17, comprenant une étape (a2) de fabrication du composé de formule (VII), préalable à l'étape (b1), par mise en réaction d'un composé de formule (X) suivante : 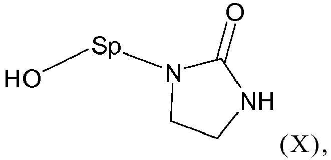 dans laquelle Sp est tel que défini à la revendication 1 ou 2,
avec un agent permettant la formation du groupe nucléofuge Z.

19. Procédé selon la revendication précédente, dans lequel ledit agent est le chlorure de thionyle.

20. Procédé selon la revendication 18 ou 19, dans lequel l'étape (a2) est réalisée en l'absence ou en présence d'au moins un solvant S₄, préférentiellement un solvant chloré, plus préférentiellement le dichlorométhane.

21. Procédé selon l'une quelconque des revendications 18 à 20, dans lequel l'étape (a2) est immédiatement suivie d'une étape (a3) de récupération du composé de formule (VII), préférentiellement par purification au toluène, plus préférentiellement par cristallisation du composé de formule (VII) dans le toluène.

## Patentansprüche

1. Zusammensetzung der folgenden Formel (I): 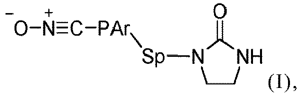 in der:
- PAr eine polyaromatische Gruppe bezeichnet, die mindestens zwei kondensierte aromatische Kohlenwasserstoffzyklen umfasst, wobei jeder der kondensierten aromatischen Zyklen eventuell durch eine oder mehrere identische oder unterschiedliche, voneinander unabhängige, aliphatische oder aromatische, lineare, verzweigte oder zyklische Kohlenstoffketten substituiert ist, die eventuell durch ein oder mehrere Heteroatome substituiert oder unterbrochen sind;
- Sp ein Atom oder eine Gruppe von Atomen repräsentiert.

2. Zusammensetzung nach Anspruch 1, **dadurch gekennzeichnet, dass** die Sp-Gruppe eine lineare oder verzweigte AlkylenKette mit C₁-C₂₄, vorzugsweise C₁-C₁₀, noch bevorzugter C₁-C₆ ist, die eventuell durch ein oder mehrere Stickstoff-, Schwefel- oder Sauerstoffatome unterbrochen ist.

3. Zusammensetzung nach Anspruch 1 oder 2, **dadurch gekennzeichnet, dass** sie von der folgenden Formel (II) ist: 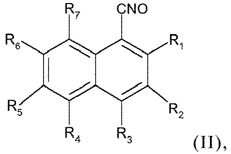 in der:
- eine aus R₁ bis R₇ ausgewählte Gruppierung die Gruppe der folgenden Formel (III) bezeichnet: 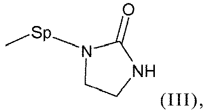
in der Sp wie in Anspruch 1 oder 2 definiert ist,
die weiteren sechs Gruppierungen, identisch oder unterschiedlich, unabhängig voneinander ein Wasserstoffatom oder eine aliphatische oder aromatische, lineare, verzweigte oder zyklische Kohlenstoff-Kette repräsentieren, die eventuell durch ein oder mehrere Heteroatome substituiert oder unterbrochen ist.

4. Zusammensetzung nach einem der vorstehenden Ansprüche, **dadurch gekennzeichnet, dass** sie aus einer Zusammensetzung der folgenden Formel (IV) ausgewählt ist: 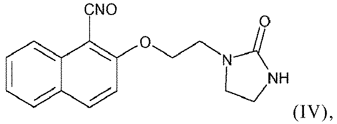 und einer Zusammensetzung der folgenden Formel (V): 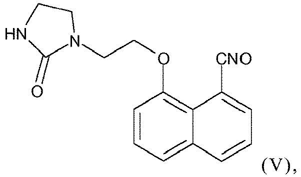 vorzugsweise die Zusammensetzung der Formel (IV).

5. Verfahren zur Synthese der Zusammensetzung der Formel (I) nach einem der vorstehenden Ansprüche, die folgenden aufeinanderfolgenden Schritte umfassend:
(b1) die Reaktion einer Zusammensetzung der folgenden Formel (VI): 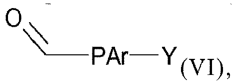 in der PAr wie in Anspruch 1 definiert ist und Y eine nucleophile Gruppe repräsentiert,
mit einer Zusammensetzung der folgenden Formel (VII): 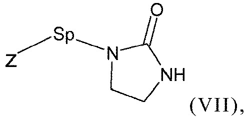 in der Sp wie in Anspruch 1 oder 2 definiert ist und Z eine nucleofuge Gruppe repräsentiert;
in Gegenwart mindestens eines polaren Lösungsmittels S₁, mindestens einer Base, bei einer Temperatur T₁, die von 70 bis 150 °C reicht,
zur Bildung einer Zusammensetzung der folgenden Formel (VIII): 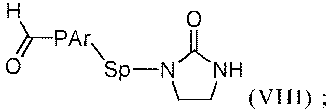
(b2) die Reaktion der Zusammensetzung der Formel (VIII) mit einer wässrigen Hydroxylamin-Lösung bei einer Temperatur T₂, die von 30 bis 70 °C reicht, um eine Oxim-Zusammensetzung der folgenden Formel (IX) zu erhalten: 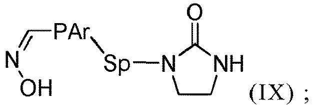
(c) einen Schritt zur Wiedergewinnung der Oxim-Zusammensetzung der Formel (IX);
(d) einen Oxidationsschritt der Oxim-Zusammensetzung der Formel (IX) mit einem Oxidationsmittel in Gegenwart von mindestens einem organischen Lösungsmittel S₂.

6. Verfahren nach Anspruch 5, wobei die beiden Schritte (b1) und (b2) durch einen Isolations- und Reinigungsschritt der Zusammensetzung der Formel (VIII) getrennt sind.

7. Verfahren nach Anspruch 5, wobei die beiden Schritte (b1) und (b2) gemäß einer Eintopfsynthese durchgeführt werden.

8. Verfahren nach einem der Ansprüche 5 bis 7, wobei die Y-Gruppe aus den primären oder sekundären Hydroxyl-, Thiol- und Amin-Funktionen ausgewählt wird.

9. Verfahren nach einem der Ansprüche 5 bis 8, wobei die Z-Gruppe aus Chlor, Brom, Iod, der Mesylatgruppe, der Tosylatgruppe, der Acetatgruppe und der Trifluormethylsulfonatgruppe ausgewählt wird.

10. Verfahren nach einem der Ansprüche 5 bis 9, wobei das polare Lösungsmittel S₁ ein in Wasser mischbares polares, vorzugsweise ein protisches Lösungsmittel, bevorzugter ein alkoholisches protisches Lösungsmittel ist.

11. Verfahren nach einem der Ansprüche 5 bis 10, wobei die Zusammensetzung der Formel (VI) von 5 bis 40 Gew.-%, vorzugsweise von 10 bis 30 Gew.-% in Bezug auf das Gewicht des Lösungsmittels repräsentiert.

12. Verfahren nach einem der Ansprüche 5 bis 11, wobei die Base aus den alkalischen Alkoholaten, den alkalischen Karbonaten, den erdalkalischen Karbonaten, den alkalischen Hydroxiden, den erdalkalischen Hydroxiden und deren Gemischen ausgewählt wird.

13. Verfahren nach einem der Ansprüche 5 bis 12, wobei die Base aus dem Natriummethanolat, dem Kaliumkarbonat und Soda, vorzugsweise dem Kaliumkarbonat ausgewählt wird.

14. Verfahren nach einem der Ansprüche 5 bis 13, wobei die molare Basenmenge von 1,5 bis 8 molaren Äquivalenten, vorzugsweise von 2 bis 6 molaren Äquivalenten in Bezug auf die molare Menge der Zusammensetzung der Formel (VI) ist.

15. Verfahren nach einem der Ansprüche 5 bis 14, wobei die Beigabe der wässrigen Hydroxylaminlösung durchgeführt wird, wenn die Umwandlung der Zusammensetzung der Formel (VI) mindestens 70 Gew.-% beträgt.

16. Verfahren nach einem der Ansprüche 5 bis 15, wobei das Oxidationsmittel aus Natriumhypochlorit, N-Bromsuccinimid in Gegenwart einer Base, N-Chlorsuccinimid in Gegenwart einer Base, Wasserstoffperoxid, in Gegenwart eines Katalysators, vorzugsweise Natriumhypochlorit ausgewählt wird.

17. Verfahren nach einem der Ansprüche 5 bis 16, wobei das organische Lösungsmittel S₂ ein organisches Lösungsmittel ist, das aus den chlorierten Lösungsmitteln und vom Typ Ester, Ether und Alkohol, ausgewählt wird, bevorzugter aus dem Dichlormethan, Ethylacetat, Butylacetat, Diethylether, Isopropanol und Ethanol, noch bevorzugter aus dem Ethylacetat und dem Butylacetat ausgewählt wird.

18. Verfahren nach einem der Ansprüche 5 bis 17, einen Schritt (a2) zur Herstellung der Zusammensetzung der Formel (VII) vor dem Schritt (b1), durch Reagieren einer Zusammensetzung der folgenden Formel (X) umfassend: 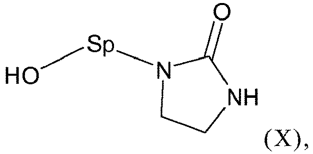 in der Sp wie in Anspruch 1 oder 2 definiert ist,
mit einem Mittel, das die Bildung der nucleofugen Gruppe Z ermöglicht.

19. Verfahren nach dem vorstehenden Anspruch, wobei das Mittel das Thionylchlorid ist.

20. Verfahren nach Anspruch 18 oder 19, wobei der Schritt (a2) in Abwesenheit oder in Gegenwart mindestens eines Lösungsmittels S₄, vorzugsweise eines chlorierten Lösungsmittels, bevorzugter des Dichlormethans durchgeführt wird.

21. Verfahren nach einem der Ansprüche 18 bis 20, wobei auf den Schritt (a2) unmittelbar ein Schritt (a3) der Wiedergewinnung der Zusammensetzung der Formel (VII), vorzugsweise durch Reinigung mit Toluen, bevorzugter durch Kristallisation der Zusammensetzung der Formel (VII) in dem Toluen folgt.

## Claims

1. A compound of formula (I) below: 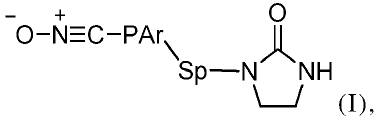 wherein:
- PAr denotes a polyaromatic group comprising at least two fused aromatic hydrocarbon rings, each of said fused aromatic rings being optionally substituted by one or more carbon-based chains, which are identical or different, independent of one another, aliphatic or aromatic, linear, branched or cyclic, and optionally substituted or interrupted by one or more heteroatoms;
- Sp represents an atom or a group of atoms.

2. The compound as claimed in claim 1, **characterized in that** the Sp group is a linear or branched C₁-C₂₄, preferentially C₁-C₁₀, more preferentially C₁-C₆, alkylene chain optionally interrupted by one or more nitrogen, sulfur or oxygen atoms.

3. The compound as claimed in claim 1 or 2, **characterized in that** it is of formula (II) below: 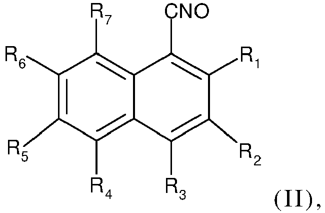 wherein:
- a group selected from R₁ to R₇ denotes the group of formula (III) below: 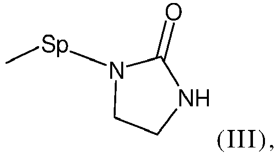
wherein Sp is as defined in claim 1 or 2,
the other six groups, which are identical or different, representing, independently of one another, a hydrogen atom or an aliphatic or aromatic, linear, branched or cyclic carbon-based chain which is optionally substituted or interrupted by one or more heteroatoms.

4. The compound as claimed in any one of the preceding claims, **characterized in that** it is selected from a compound of formula (IV) below: 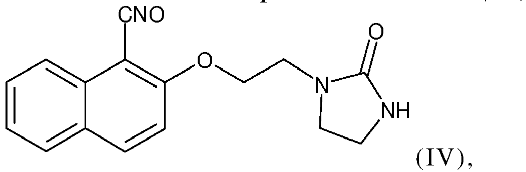 and a compound of formula (V) below: 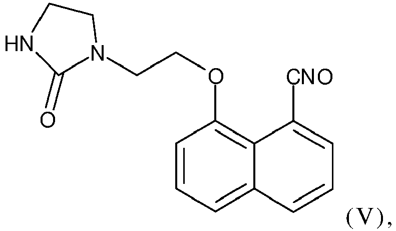 preferably the compound of formula (IV).

5. A process for synthesizing the compound of formula (I) as defined in any one of the preceding claims, comprising the following successive steps:
(b1) the reaction of a compound of formula (VI) below: 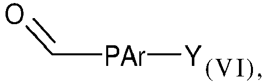 wherein PAr is as defined in claim 1 and Y represents a nucleophilic group,
with a compound of formula (VII) below: 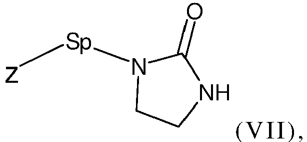 wherein Sp is as defined in claim 1 or 2, and Z represents a nucleofuge group;
in the presence of at least one polar solvent S₁, and of at least one base, at a temperature T₁ ranging from 70 to 150°C,
in order to form a compound of formula (VIII) below: 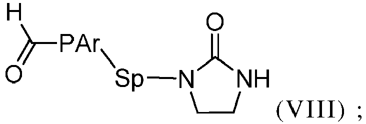
(b2) the reaction of said compound of formula (VIII) with an aqueous hydroxylamine solution at a temperature T₂ ranging from 30 to 70°C, in order to obtain an oxime compound of formula (IX) below: 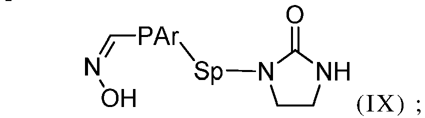
(c) a step of recovery of said oxime compound of formula (IX);
(d) a step of oxidation of the oxime compound of formula (IX) with an oxidizing agent, in the presence of at least one organic solvent S₂.

6. The process as claimed in claim 5, wherein the two steps (b1) and (b2) are separated by a step of isolation and purification of the compound of formula (VIII).

7. The process as claimed in claim 5, wherein the two steps (b1) and (b2) are carried out according to a one-pot synthesis.

8. The process as claimed in any one of claims 5 to 7, wherein the Y group is selected from hydroxyl, thiol and primary or secondary amine functions.

9. The process as claimed in any one of claims 5 to 8, wherein the Z group is selected from chlorine, bromine, iodine, the mesylate group, the tosylate group, the acetate group and the trifluoromethylsulfonate group.

10. The process as claimed in any one of claims 5 to 9, wherein the polar solvent S₁ is a water-miscible polar solvent, preferably a protic solvent, more preferentially an alcoholic protic solvent.

11. The process as claimed in any one of claims 5 to 10, wherein the compound of formula (VI) represents from 5 to 40% by weight, preferably from 10 to 30% by weight, relative to the weight of the solvent.

12. The process as claimed in any one of claims 5 to 11, wherein the base is selected from alkali metal alkoxides, alkali metal carbonates, alkaline earth metal carbonates, alkali metal hydroxides, alkaline earth metal hydroxides and mixtures thereof.

13. The process as claimed in any one of claims 5 to 12, wherein the base is selected from sodium methoxide, potassium carbonate and sodium hydroxide, preferably potassium carbonate.

14. The process as claimed in any one of claims 5 to 13, wherein the molar amount of base is from 1.5 to 8 molar equivalents, preferably from 2 to 6 molar equivalents, relative to the molar amount of compound of formula (VI).

15. The process as claimed in any one of claims 5 to 14, wherein the addition of the aqueous hydroxylamine solution is carried out when the conversion of the compound of formula (VI) is at least 70% by weight.

16. The process as claimed in any one of claims 5 to 15, wherein the oxidizing agent is selected from sodium hypochlorite, N-bromosuccinimide in the presence of a base, N-chlorosuccinimide in the presence of a base, and aqueous hydrogen peroxide solution in the presence of a catalyst, preferably sodium hypochlorite.

17. The process as claimed in any one of claims 5 to 16, wherein the organic solvent S₂ is an organic solvent selected from chlorinated solvents and solvents of ester, ether and alcohol type, more preferably selected from dichloromethane, ethyl acetate, butyl acetate, diethyl ether, isopropanol and ethanol, more preferably still selected from ethyl acetate and butyl acetate.

18. The process as claimed in any one of claims 5 to 17, comprising a step (a2) of manufacture of the compound of formula (VII), prior to step (b1), by reacting a compound of formula (X) below: 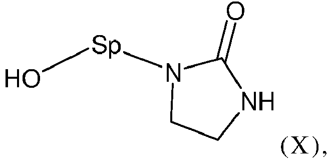 wherein Sp is as defined in claim 1 or 2,
with an agent which makes possible the formation of the nucleofuge group Z.

19. The process as claimed in the preceding claim, wherein said agent is thionyl chloride.

20. The process as claimed in claim 18 or 19, wherein step (a2) is carried out in the absence or in the presence of at least one solvent S₄, preferably a chlorinated solvent, more preferentially dichloromethane.

21. The process as claimed in any one of claims 18 to 20, wherein step (a2) is immediately followed by a step (a3) of recovery of the compound of formula (VII), preferentially by purification with toluene, more preferentially by crystallization of the compound of formula (VII) from toluene.
